# EUROPEAN PATENT APPLICATION

(11) **EP 1 748 071 A1**
(43) Date of publication of application: **31.01.2007**
(21) Application number: 05741599.4
(22) Date of filing: 19.05.2005
(51) Int. Cl.: C12N 15/09, C12P 21/02

(54) **PROCESS FOR PRODUCING POLYPEPTIDE**

(30) Priority: 21.05.2004 JP 2004152598; 10.03.2005 JP 2005067984
(71) Applicant: Takara Bio, Inc., Otsu-shi, Shiga 520-2193 (JP)
(72) Inventor: SHODAI, Toshihiro, Otsu-shi, Shiga 520-2193 (JP); KOBORI, Hiroshi, Otsu-shi, Shiga 520-2193 (JP); SAGARA, Takehiro, Otsu-shi, Shiga 520-2193 (JP); ENDO, Hiroshi, Otsu-shi, Shiga 520-2193 (JP); TAKAKURA, Hikaru, Otsu-shi, Shiga 520-2193 (JP); TOMONO, Jun, Okayama 710-0845 (JP); SAGAWA, Hiroaki, Otsu-shi, Shiga 520-2193 (JP); MUKAI, Hiroyuki, Otsu-shi, Shiga 520-2193 (JP); KATO, Ikunoshin, Otsu-shi, Shiga 520-2193 (JP)
(74) Representative: Schnappauf, Georg
(86) International application number: PCT/JP2005/009184
(87) International publication number: WO 2005/113768

(57) **Abstract**

A process for producing a target protein at low temperature, comprising inducing expression of not only a vector having introduced therein a gene coding for the target protein but also a vector having a chaperone gene introduced therein.

## Description

### Technical Field

The present invention relates to a method for producing a protein of interest under low-temperature conditions by expression of a vector having a gene encoding the protein of interest being incorporated and a vector having a chaperone gene being incorporated.

### Background Art

Recently, analyses of genomes of various organisms are being completed, and are considered to be shifted to exhaustive functional analyses of proteins as expression products of genes in the future. Studies to aid in elucidating biological phenomena by clarifying properties of individual proteins and exhaustively analyzing interactions between proteins are rapidly increasing. On the other hand, a great interest is had in determination of three-dimensional structures of intracellular receptor proteins which specifically bind to various physiologically active substances to transmit the actions. This is because active substances that bind to the receptor proteins can be candidate substances for novel medicines. Thus, attention is paid concerning screening for novel medicines. For determining a property of such a protein, a general method comprises incorporation of the corresponding gene into a vector gene, transformation of a host such as a bacterium, a yeast or an insect cell, and examination of a property of a recombinant protein obtained by expression.

When an accurate property of a protein is to be estimated, it is very important whether or not the protein is folded into a proper tertiary structure. However, if a protein derived from a heterologous organism is to be prepared according to a protein expression method using a host expression system as described above, one may often encounter a case where only an abnormal protein having a different tertiary structure is obtained due to abnormal folding of the protein. Such a protein forms an aggregate called an inclusion body in a host. Formation of an inclusion body is advantageous in that the expressed protein is protected from degradation by proteolytic enzymes in the host cell, and can be readily separated from the cell by centrifugation. However, it is necessary to solubilize the inclusion body by denaturation and then regenerate (refold) it in order to obtain a biologically active protein of interest. The procedure of solubilization/regeneration is empirically conducted while repeating trial and error for each protein. Satisfactory yield is not achieved in many cases and, moreover, regeneration is not necessarily possible. Furthermore, high expression levels are not achieved for not a few heterologous proteins because the proteins are degraded by proteases in Escherichia coli. It can hardly be said that a means to solve the problem of insolubilization or degradation of such expression products has been sufficiently established. At present, production of a biologically active protein in large quantities using a host expression system as described above is not necessarily successful. For solving the problem, co-expression with a chaperone or the like has been attempted, and some reports have been made (for example, see Patent Documents 1 to 3) .

DnaK, DnaJ and GrpE are chaperones which cooperatively function in folding of proteins. It is considered as follows. First, when DnaJ binds to an unfolded protein as a substrate, ATP on DnaK is hydrolyzed to form a complex of the unfolded protein/DnaJ/DnaK (ADP-binding type). Then, GrpE causes ADP/ATP exchange to release the substrate protein from the complex (for example, see Non-patent Document 1). Trigger Factor is one of molecular chaperones involved in protein folding, and has an activity of catalyzing a cis-trans isomerization reaction of a peptide bond on the N-terminal side of a proline residue among amino acids in a target protein during folding in a cell (a PPIase activity).

It is known in many cases that co-expression of foreign proteins that are insolubilized in Escherichia coli with GroEL and GroES resulted in successful solubilization of the foreign proteins. Examples thereof include tyrosine kinase (for example, see Non-patent Documents 2 and 3), glutamate racemase (for example, see Non-patent Document 4) and dihydrofolate reductase (for example, see Non-patent Document 5). Furthermore, increased solubility of human growth hormone due to co-expression with DnaK (for example, see Non-patent Document 6), solubilization of transglutaminase due to co-expression with DnaJ (for example, Non-patent Document 4) and solubilization of tyrosine kinase due to co-expression with DnaK, DnaJ and GrpE (for example, see Non-patent Document 2) are known.

An attempt has been made to solubilize a protein that is insolubilized in Escherichia coli by expressing it as a fusion protein with a chaperone or the like. For example, success in solubilization of mouse anti-chicken lysozyme Fab antibody fragment by expressing it as a fusion protein with TcFKBP18, a chaperone from an archaebacterium, is known (see Patent Document 7).

However, problems concerning expression or folding of all proteins have not been solved by the above-mentioned methods. Thus, establishment of a method for efficiently producing a protein has been strongly desired.

If a culture temperature for Escherichia coli cells during the logarithmic growth phase is lowered from 37°C to 10-20°C, growth of the Escherichia coli cells is temporarily arrested, during which expression of a group of proteins called cold shock proteins is induced. The proteins are divided into two groups according to the induction levels: a group I (10-fold or more) and a group II (less than 10-fold). Proteins in the group I include CspA, CspB, CspG and CsdA (for example, see Non-patent Documents 7 and 8). Among these, the expression level of CspA (for example, see Patent Document 5) reaches 13% of the total cellular protein 1.5 hours after temperature shift from 37°C to 10°C (for example, see Non-patent Document 9). Then, attempts have been made to utilize the promoter for the cspA gene for production of a recombinant protein at a low temperature.
Regarding a system for expressing a recombinant protein under low-temperature conditions using the cspA gene, the following effectiveness has been shown in addition to the above-mentioned highly efficient transcription initiation by the promoter for the gene at a low temperature.
(1) If mRNA that is transcribed from the cspA gene and capable of being translated does not encode a functional CspA protein (specifically, if it encodes only a portion of the N-terminal sequence of the CspA protein), such mRNA inhibits expression of other Escherichia coli proteins including cold shock proteins for a long period of time. During this period, the mRNA is preferentially translated (for example, see Non-patent Document 7 and Patent Document 6). This phenomenon is called LACE (low temperature-dependent antibiotic effect of truncated cspA expression) effect.
(2) A sequence consisting of 15 nucleotides called a downstream box is located 12 nucleotides downstream of the initiation codon of the cspA gene. This sequence enables the high translation efficiency under low-temperature conditions.
(3) A 5'-untranslated region consisting of 159 nucleotides is located between the transcription initiation site and the initiation codon in the mRNA for the cspA gene. This region has a negative effect on the expression of CspA at 37°C and a positive effect under low-temperature conditions.
   In particular, the phenomenon as described in (1) above suggests the feasibility of specific expression of only a protein of interest utilizing the cspA gene. Thus, it is expected that the system can be applied to production of highly pure recombinant proteins or preparation of isotope-labeled proteins for structural analyses.
   However, the above-mentioned recombinant protein expression system under low-temperature conditions still cannot be applied to all recombinant proteins. Respective proteins have intrinsic molecular weights, isoelectric points and amino acid compositions, and need to form unique higher order structures for exerting the functions. There are proteins for which sufficient expression levels cannot be achieved or active proteins cannot be obtained even if the above-mentioned expression system is used.

Patent Document 1: JP-A 11-9274
Patent Document 2: JP-A 2000-255702
Patent Document 3: JP-A 2000-189163
Patent Document 4: JP-A 8-308564
Patent Document 5: WO 90/09447
Patent Document 6: WO 98/27220
Patent Document 7: WO 2004/001041
Non-patent Document 1: Proc. Natl. Acad. Sci. USA, 91:10345-10349 (1994)
Non-patent Document 2: Cell Mol. Biol., 40:635-644 (1994)
Non-patent Document 3: Proc. Natl. Acad. Sci. USA, 92:1048-1052 (1995)
Non-patent Document 4: J. Biochem., 117:495-498 (1995)
Non-patent Document 5: Protein. Eng., 7:925-931 (1994)
Non-patent Document 6: Biotechnol., 10:301-304 (1992)
Non-patent Document 7: J. Bacteriol., 178:4919-4925 (1996)
Non-patent Document 8: J. Bacteriol., 178:2994-2997 (1996)
Non-patent Document 9: Proc. Natl. Acad. Sci. USA, 87:283-287 (1990)

### Disclosure of Invention

### Problems to be Solved by the Invention

The main object of the present invention is to provide, in view of the above-mentioned prior art, a method for efficiently producing a polypeptide of interest retaining its activity.

### Means to Solve the Problems

As a result of intensive studies, the present inventors have found that a protein for which it has been difficult to express a soluble protein according to a conventional method can be expressed as an active soluble protein by enhancing expression of a chaperone gene in a host to be used upon expression of a gene encoding a polypeptide or protein of interest using a recombinant protein expression system under low-temperature conditions.
Furthermore, the present inventors have found that expression of a polypeptide or protein of interest as a fusion protein with a chaperone according to the above-mentioned method is particularly effective in solubilization of the protein of interest. The effect of the method in solubilization of a protein of interest is surprising and unexpected from the effect of a conventional method in which a protein is expressed as a fusion protein with a chaperone or a method in which a protein of interest is expressed using a recombinant protein expression system under low-temperature conditions.
For example, the present invention provides a method for efficiently expressing a polypeptide of interest by transferring a vector having a gene of interest being incorporated and a vector having a chaperone gene being incorporated into the same host and inducing expression of both genes.

The first aspect of the present invention relates to a method for producing a polypeptide, the method comprising exposing a host having a gene encoding a desired polypeptide being transferred into the host to low-temperature conditions to induce expression of the polypeptide, wherein expression of a gene encoding a chaperone is enhanced in the host.
According to the first aspect, expression.of the gene encoding a chaperone may be enhanced, for example, by: induction of expression of a chaperone gene of the host; modification of a chaperone gene on a chromosome of the host; transfer of a chaperone gene into the host; or use of a host in which expression of a chaperone gene is enhanced.
According to the first aspect, a gene encoding a chaperone selected from the group consisting of DnaK, DnaJ, GrpE, GroEL, GroES and Trigger Factor may be preferably used.
According to the first aspect, the gene encoding a desired polypeptide being transferred into the host may be linked downstream of a DNA encoding a 5'-untranslated region derived from an mRNA for a cold shock protein gene. The cold shock protein gene is exemplified by Escherichia coli cspA gene.
According to the first aspect, the DNA encoding a desired polypeptide and the gene encoding a chaperone may be transferred into the host using vector (s). The DNA encoding a desired polypeptide and the gene encoding a chaperone may be linked to each other so that a fusion protein of the desired polypeptide and the chaperone is encoded. The desired polypeptide is exemplified by Rous-associated virus 2 (RAV-2) reverse transcriptase α subunit, RAV-2 β subunit, DNase or human Dicer PAZ domain polypeptide.
The host used according to the first aspect is exemplified by Escherichia coli.

The second aspect of the present invention relates to a set of plasmid vectors used for production of a desired polypeptide, comprising:
(1) a first vector having, downstream of a promoter:
   (a) a DNA encoding a 5'-untranslated region derived from an mRNA for a cold shock protein gene; and
   (b) a restriction enzyme recognition sequence that can be used for inserting a gene encoding a desired polypeptide and is located downstream of the DNA of (a); and
(2) a second vector having a gene encoding a chaperone,
   wherein replication origins of the vectors of (1) and (2) are selected so that incompatibility is not exerted.
According to the second aspect, for example, the first vector may contain a DNA encoding a 5'-untranslated region derived from an mRNA for Escherichia coli cspA gene, and the second vector may contain a gene encoding a chaperone selected from the group consisting of DnaK, DnaJ, GrpE, GroEL, GroES and Trigger Factor. Plasmids that are capable of replicating in Escherichia coli may be used as the vectors.

The third aspect of the present invention relates to an expression vector having, downstream of a promoter:
(a) a DNA encoding a 5'-untranslated region derived from an mRNA for a cold shock protein gene;
(b) a DNA having a restriction enzyme recognition sequence that can be used for inserting a gene encoding a desired polypeptide and is located downstream of the DNA of (a); and
(c) a gene encoding a chaperone.
The expression vector of the third aspect is exemplified by one that contains a DNA encoding a 5'-untranslated region derived from an mRNA for Escherichia coli cspA gene, or one that contains a gene encoding a chaperone selected from the group consisting of DnaK, DnaJ, GrpE, GroEL, GroES and Trigger Factor.
According to the third aspect, the restriction enzyme recognition sequence that can be used for inserting a gene encoding a desired polypeptide may be located at a position at which the gene encoding a desired polypeptide can be inserted so that the desired polypeptide is expressed as a fusion protein with the chaperone.
The expression vector may be a plasmid capable of replicating in Escherichia coli.

### Effects of the Invention

According to the method of the present invention, it is possible to obtain a considerable amount of a polypeptide that has been conventionally difficult to be expressed while retaining its activity.

### Brief Description of Drawings

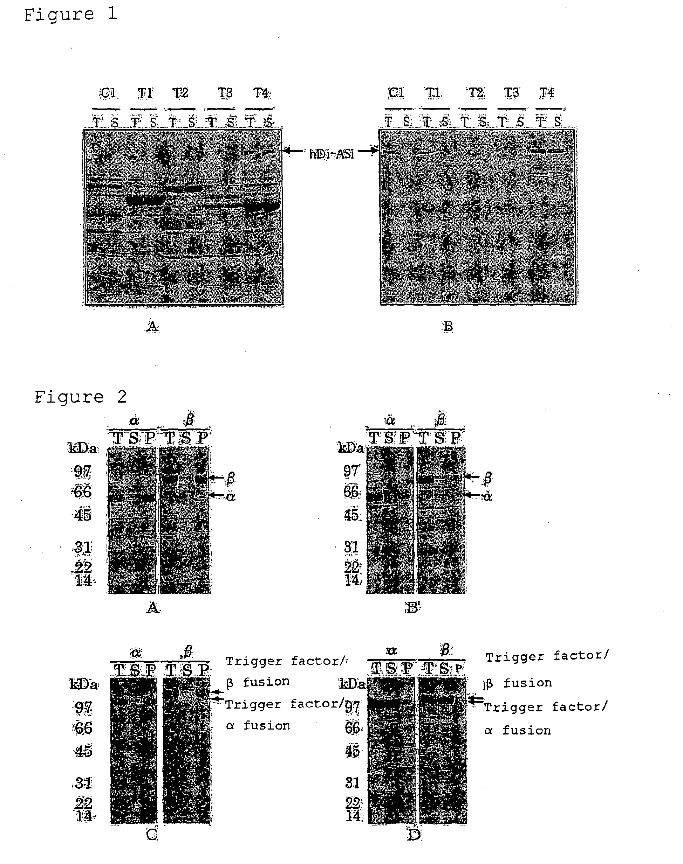

Figure 1 shows examination results of expression of hDi-ASI by co-expression. "A" and "B" show results of CBB staining and antibody staining, respectively. In the figure, "T" represents a cell extract fraction and "S" represents a soluble fraction.
Figure 2 shows examination results of expression of a fusion protein of RTaseα or RTaseβ and Trigger Factor. "A" and "B" show results of sole expression and co-expression, respectively, and "C" and "D" show results of fusion expression in T7 promoter expression system and fusion expression in cold shock expression system, respectively. In the figure, "α" represents RAV-2 RTaseα, "β" represents RAV-2 RTaseβ, "T" represents a cell extract fraction, "S" represents a soluble fraction, and: "P" represents an insoluble fraction.

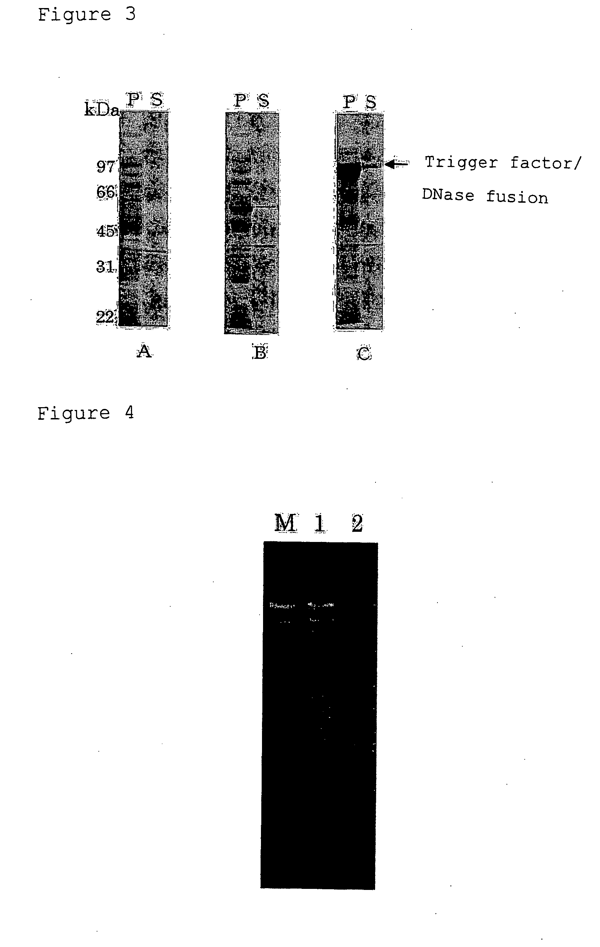

Figure 3 shows examination results of expression of a fusion protein of DNase and Trigger Factor. "A", "B" and "C" show results of sole expression system, co-expression system and fusion expression system, respectively. In the figure, "S" represents a soluble fraction and "P" represents an insoluble fraction.
Figure 4 shows results of DNase activity measurement. In the figure, "M" represents substrate alone, "1" represents results of DNase activity measurement using a sonication soluble fraction as a control, and "2" represents results of DNase activity measurement using a sonication soluble fraction of fusion expression system.

### Best Mode for Carrying Out the Invention

Hereinafter, the present invention will be described.

### (1) Cold shock vector

A vector containing a gene encoding a desired polypeptide used according to the present invention is one in which expression of the polypeptide is induced by shifting the cultivation temperature of the host to one lower than the normal growth temperature, i.e., by cold shock. It is used as an expression vector according to the present invention. As used herein, the term "low temperature" refers to a temperature lower than the normal growth temperature of the host. Hereinafter, the above-mentioned vector may be referred to as a cold shock vector. A system for expressing a desired polypeptide utilizing such a vector may be referred to as cold shock expression system. One having a DNA encoding a 5'-untranslated region derived from an mRNA for a cold shock protein gene and a gene encoding a desired polypeptide linked downstream of the DNA can be used as such a vector. As used herein, the term "downstream" refers to a downstream position in relation to the transcription direction.
Although it is not intended to limit the present invention, in a preferred embodiment, a vector containing a gene encoding a desired polypeptide comprises the following elements:
(A) a promoter that acts in a host to be used;
(B) a regulatory region for regulating the action of the promoter of (A); and
(C) a portion encoding a 5'-untranslated region derived from an mRNA for a cold shock protein gene or a region in which at least one nucleotide is substituted, deleted, inserted or added in the untranslated region.

### Details are described below.

There is no specific limitation concerning the promoter of (A) as long as it has an activity of initiating transcription of RNA in a host to be used. Specifically, it is an arbitrary promoter that can be utilized as a cold-responsive promoter by using it in combination the portion encoding a 5'-untranslated region derived from an mRNA for a cold shock protein gene of (C).
There is no specific limitation concerning the regulatory gene of (B) as long as it can be used to regulate expression of a gene located downstream of the promoter of (A). For example, translation of a protein of interest from a gene downstream of the promoter can be inhibited by incorporating into the vector a region from which an RNA complementary to an mRNA transcribed from the promoter (antisense RNA) is transcribed. Expression of a polypeptide of interest can be regulated by transcribing the antisense RNA under control of an appropriate promoter different from the promoter of (A). Operators present in expression-regulatory regions of various genes may be utilized. For example, the lac operator derived from the Escherichia coli lactose operon can be used according to the present invention. A promoter can be allowed to act by canceling the function of the lac operator using an appropriate inducer such as lactose or a structural analog thereof (preferably, isopropyl-β-D-thiogalactoside (IPTG)). Such an operator sequence is usually placed downstream of a promoter and near a transcription initiation site.
The portion encoding a 5'-untranslated region derived from an mRNA for a cold shock protein of (C) is a portion that encodes a region of an mRNA 5' to the initiation codon. Such portions have been characteristically found in Escherichia coli cold shock protein genes (cspA, cspB, cspG and csdA) (J. Bacteriol., 178:4919-4925 (1996); J. Bacteriol., 178:2994-2997 (1996)). A portion of 100 nucleotides or more from the 5' end in the mRNA transcribed from such a gene is not translated into a protein. This portion is important for cold response of gene expression. If this 5'-untranslated region is attached to an mRNA for an arbitrary protein at its 5' end, translation from the mRNA into a protein takes place under low-temperature conditions. One or more nucleotide(s) may be substituted, deleted, inserted or added in the nucleotide sequence of the 5'-untranslated region derived from an mRNA for a cold shock protein as long as the function is retained.

As used herein, "a region" refers to an area of a nucleic acid (DNA or RNA). As used herein, "a 5'-untranslated region of an mRNA" refers to a region that does not encode a protein, and is located on the 5' side of an mRNA synthesized as a result of transcription from a DNA. Hereinafter, the region is referred to as "a 5'-UTR (5'-Untranslated Region)". Unless otherwise noted, the 5'-UTR refers to a 5'-untranslated region of mRNA for the Escherichia coli cspA gene or a modification thereof.

Portions encoding 5'-UTRs derived from the cold shock protein genes as listed above can be used for the vector of the present invention. In particular, one derived from the Escherichia coli cspA gene can be preferably used. Furthermore, one in which the nucleotide sequence is partially modified may be used as long as it can contribute to cold-specific expression of a polypeptide. For example, one in which the nucleotide sequence of the region is modified by incorporating an operator as described above with respect to (B) may be used. Alternatively, the portion encoding a 5'-UTR of a cold shock protein gene may be placed between the promoter of (A) and an initiation codon of a gene encoding a polypeptide to be expressed. An operator may be incorporated in the portion.

It is possible to increase expression efficiency by including downstream of a 5'-untranslated region, in addition to the above-mentioned elements, a nucleotide sequence complementary to an anti-downstream box sequence in ribosomal RNA of a host to be used. For example, in case of Escherichia coli, an anti-downstream box sequence is present from position 1467 to position 1481 in 16S ribosomal RNA. It is possible to use a region encoding an N-terminal peptide of a cold shock protein which contains a nucleotide sequence highly complementary to this sequence. A vector in which a transcription termination sequence (terminator) is placed downstream of a gene for a protein of interest is advantageous to high expression of the protein of interest because the stability of the vector is increased.

The vector of the present invention may be any one of commonly used vectors (e.g., plasmid, phage or virus vectors) as long as it can be used to achieve the object as a vector. Regarding a region contained in addition to the above-mentioned elements, the vector of the present invention may have, for example, a replication origin, a drug-resistance gene used as a selectable marker, or a regulatory gene necessary for a function of an operator (e.g., the lacI^{q} gene for the lac operator). It does not create inconvenience if the vector of the present invention is integrated into a genomic DNA in a host after it is transferred into the host.

Construction of a plasmid vector is specifically described below. Unless otherwise noted, Escherichia coli CspA protein, a region of a gene involved in expression of the protein, and a promoter region in the gene are herein referred to as "CspA", "cspA gene" and "cspA promoter", respectively. In the nucleotide sequence of native cspA gene (SEQ ID NO:1) which is registered in the GeneBank gene database under accession no. M30139 and available to the public, the major transcription initiation point (+1) is located at nucleotide 426, the SD sequence (ribosome binding sequence) is located from nucleotide 609 to nucleotide 611, the initiation codon for CspA is located from nucleotide 621 to nucleotide 623, and the termination codon for CspA is located at nucleotide 832 to nucleotide 834, respectively. Then, the portion from nucleotide 462 to nucleotide 620 in the sequence encodes 5'-UTR. The partially modified 5'-UTR is exemplified by one described in WO 99/27117. The 5'-UTR region contained in the vector pCold08NC2 used in Examples is an example thereof. The nucleotide sequence is shown in SEQ ID NO:2.

A nucleotide sequence highly complementary to an anti-downstream box sequence which is present in 16S ribosomal RNA (downstream box sequence) may be incorporated into the vector for increasing expression efficiency of a gene of interest. A downstream box sequence which is present in a region encoding an N-terminal portion of Escherichia coli CspA has only 67% complementarity to the above-mentioned anti-downstream box sequence. Use of a nucleotide sequence having higher complementarity than the above (preferably 80% or more, more preferably 100% (Perfect DB sequence)) enables expression of a gene linked downstream with higher efficiency.
Furthermore, a nucleotide sequence encoding a tag sequence, which is a peptide for facilitating purification of an expressed gene product of interest, or a nucleotide sequence encoding a protease recognition amino acid sequence, which is utilized for removal of an extra peptide in a gene product of interest (e.g., a tag sequence), may be incorporated into the vector.
A histidine tag (His Tag) which consists of several histidine residues, a maltose-binding protein, glutathione-S-transferase, or the like may be used as a tag sequence for purification. A polypeptide having a histidine tag being attached can be readily purified using a chelating column. As to other tag sequences, purification can be also conveniently conducted by using ligands having specific affinities. Factor Xa, thrombin enterokinase or the like can be used as a protease utilized for removal of an extra peptide. A nucleotide sequence encoding an amino acid sequence specifically cleaved with such a protease may be incorporated into the vector.

### (2) Enhancement of chaperone expression

The method for producing a polypeptide of the present invention is characterized by enhancement of expression of a gene encoding a chaperone upon expression of a gene encoding a polypeptide of interest.
According to the present invention, any protein may be used as a chaperone as long as it is a protein involved in folding of proteins. Examples thereof include DnaK, DnaJ, GrpE, GroEL, GroES and Trigger Factor from Escherichia coli.
Although it is not intended to limit the present invention, it is preferable to use a protein involved in isomerization at proline in a polypeptide such as Trigger Factor (also called peptidyl-prolyl cis-trans isomerase (PPIase)) for expression of a polypeptide using a cold shock vector. The chaperone is not limited to one derived from Escherichia coli. For example, a chaperone derived from an archaebacterium, a yeast, a microorganism or a psychrophile can be utilized.

Enhancement of a chaperone.gene expression can be accomplished by induction of expression of a gene encoding a chaperone on a chromosome, or a known technique such as modification of a chaperone gene on a chromosome (increase in copy number or insertion of a promoter), transfer of a chaperone gene into a host, or obtainment of a strain highly expressing a chaperone gene by mutagenesis of a host.
For example, if conditions under which expression of a chaperone is induced in a host are known, induction of expression of the chaperone gene on a chromosome can be induced utilizing the conditions. Modification of a chaperone gene on a chromosome can be carried out for a host chromosome using a technique of site-directed mutagenesis or gene insertion utilizing homologous recombination. For example, expression can be induced using an inducible promoter that has been inserted upstream of a chaperone-encoding gene to be induced on a host chromosome.
In another embodiment, a mutant strain of a host to be used for expression of a polypeptide in which expression of a chaperone gene is enhanced is obtained and used as a host. A mutant strain can be obtained according to a known method, for example, by treating a host microorganism with a mutagen such as an agent or ultraviolet light and then selecting a strain in which expression of a chaperone gene is enhanced.
Enhancement of expression of a gene encoding a chaperone means that the amount of the chaperone protein in the host is increased as compared with the normal amount. It is possible to confirm if expression of a chaperone gene is enhanced according to the above-mentioned procedure, for example, by measuring a chaperone protein utilizing an antibody that recognizes the chaperone, or by measuring the amount of an mRNA transcribed from the gene encoding the chaperone using a known method (e.g., RT-PCR method, Northern hybridization, or hybridization using a DNA array).
It is advantageous that expression of a chaperone and expression of a protein of interest can be independently controlled so as to optimize the amount or the timing of expression of the chaperone without decreasing the expression level of the protein of interest. For this purpose, it is preferable that a chaperone gene is placed downstream of a controllable promoter. Furthermore, it is preferable that the controllable promoter used for expression of the chaperone is different from the promoter used for expression of the protein of interest.

Expression of a chaperone gene may be enhanced by
inserting a chaperone gene into a vector and transferring it into a host. The vector may be any one of commonly used vectors (e.g., plasmid, phage or virus vectors) as long as it can be used to achieve the object as a vector.
Usually, two closely related plasmids cannot stably coexist in a single host. This phenomenon is called incompatibility. According to the present invention, if a plasmid is to be used as a vector containing a chaperone gene (hereinafter also referred to as a chaperone plasmid), it is preferable to use one having a replicon that does not exhibit incompatibility with the expression vector for a protein of interest. For example, if one having ColE1 replicon (e.g., pCold07 described in WO 99/27117) is to be used as an expression vector for a protein of interest, p15A replicon in a vector pACYC or the like may be used for a chaperone plasmid.

According to the present invention, a selectable marker gene may further be included optionally so that selection can be readily carried out upon transformation with a vector containing a chaperone gene. Such selectable marker genes include ampicillin resistance (Amp^{r}) gene, kanamycin resistance (Km^{r}) gene and chloramphenicol resistance (Cm^{r}) gene. It is desirably different from the selectable marker gene included in the expression vector for a foreign protein.

Specific examples of chaperone plasmids used according to the present invention include a plasmid pG-KJE8 which expresses DnaK/DnaJ/GrpE and GroEL/GroES, a plasmid Gro7 which expresses GroEL/GroES, a plasmid pKJE7 which expresses DnaK/DnaJ/GrpE, a plasmid pG-Tf2 which expresses GroEL/GroES and Trigger Factor, and a plasmid pTf16 which expresses Trigger Factor (all from Takara Bio).

A gene encoding a chaperone may be transferred into a host using a vector as described above, or it may be used being integrated in a host chromosome.

According to the present invention, the foreign protein to be expressed may be any protein as long as it is destabilized and/or insolubilized in the host. Such foreign proteins include interferons, interleukins, interleukin receptors, interleukin receptor antagonists, granulocyte colony-stimulating factor, granulocyte macrophage colony-stimulating factor, macrophage colony-stimulating factor, erythropoietin, thrombopoietin, leukemia inhibitory factor, stem cell growth factor, tumor necrosis factor, growth hormone, proinsulin, insulin-like growth factor, fibroblast growth factor, platelet-derived growth factor, transforming growth factor, hepatocyte growth factor, bone morphogenetic factor, nerve growth factor, ciliary neurotrophic factor, brain-derived neurotrophic factor, glial cell-derived neurotrophic factor, neurotrophin, prourokinase, tissue plasminogen activator, blood coagulation factors, protein C, glucocerebrosidase, superoxide dismutase, renin, lysozyme, P450, prochymosin, trypsin inhibitors, elastase inhibitors, lipocortin, leptin, immunoglobulins, single-chain antibodies, complement components, blood albumins, cedar pollen antigens, hypoxia-induced stress protein, protein kinase, protooncogene products, transcription regulatory factors and virus-constituting proteins.

The expression vector of the present invention is transferred into a host and subjected to expression of a protein of interest. Examples of the hosts include, but are not limited to, prokaryotes (e.g., bacteria), yeasts, fungi, plants, insect cells and mammalian cells. The characteristics of the expression vector must be matched to the host to be used. For example, if a protein is to be expressed in a mammalian cell system, it is preferable to use a promoter isolated from a genome of a mammalian cell (e.g., mouse metallothionein promoter) or a promoter isolated from a virus that multiplies in such as cell (e.g., baculovirus promoter, vaccinia virus 7.5K promoter) for the expression vector.

Among others, a prokaryote such as Escherichia coli is preferably used as a host. If a Gram-negative bacterium is to be used as a host, a protein may be expressed in cytoplasm or in the periplasmic space.

There is no specific limitation concerning the method for transferring a chaperone vector and an expression vector into a host according to the present invention, and various known methods may be used. Examples thereof include transfection according to a calcium phosphate precipitation method, electroporation, liposome fusion, nuclear injection, infection with a virus or a phage. The present invention also encompasses a host containing the expression vector of the present invention. The process of transfer into a host may be in a one-step form in which a chaperone vector and an expression vector are transferred at the same time, or in a two-step form in which an expression vector is transferred after a chaperone vector is transferred, or a chaperone vector is transferred after an expression vector is transferred. Co-transformed strains may be screened using a drug corresponding to a selectable marker gene. Expression of a foreign protein can be confirmed, for example, by Western blotting or the like.

In one aspect, the present invention relates to a set of vectors for expression of a polypeptide comprising a combination of the above-mentioned cold shock vector and the vector containing a chaperone gene. In this aspect, it is preferable to use, as a cold shock vector, one into which one can insert a gene encoding a polypeptide whose expression is desired for the object. Examples thereof include one having (a) a DNA encoding 5'-untranslated region derived from an mRNA for a cold shock protein gene; and (b) a restriction enzyme recognition sequence that can be used for inserting a gene encoding a desired polypeptide and is located downstream of the DNA of (a).

In another aspect, the present invention relates to a vector in which a chaperone-encoding gene is inserted into the cold shock vector. In this case, a transformant that can be used for expressing a polypeptide of interest can be prepared by transforming a host with a single vector.
In this aspect, a restriction enzyme recognition sequence that can be used for inserting a gene encoding a polypeptide of interest may be arranged at a position at which a gene encoding a chaperone is connected in-frame to the gene encoding a polypeptide of interest so that a fusion protein of the chaperone and the polypeptide of interest is expressed. In a fusion protein of a chaperone and a polypeptide of interest, the chaperone may be connected on the N-terminal side or the C-terminal side, or both, of the polypeptide of interest. A fusion protein may have an amino acid or a peptide as a linker between a chaperone and a polypeptide of interest. The chain length of the linker is preferably 1 to 50 amino acid(s), more preferably 3 to 40 amino acids, most preferably 5 to 30 amino acids. The amino acid sequence of the linker may be a protease recognition sequence or one in which arranged plural protease recognition sequences are inserted. Examples of such protease recognition sequences include recognition sequences for various proteases such as factor Xa, thrombin, enterokinase (all available from Takara Bio) and PreScission Protease (Amersham Biosciences). For example, the protease recognition sequence is preferably a sequence consisting of 4 to 8 amino acids.

A fusion polypeptide of a polypeptide of interest and a chaperone can be obtained by inserting a gene encoding the polypeptide of interest into the above-mentioned vector and transferring it into an appropriate host. If the fusion polypeptide has a linker containing a recognition sequence for a protease, it is possible to obtain the polypeptide of interest having been separated from the chaperone by digesting it with the protease.

### (3) Method for producing polypeptide

The method for producing a polypeptide of the present invention is carried out, for example, with the following steps.
A gene encoding a polypeptide of interest is inserted downstream of a DNA encoding a 5'-untranslated region derived from an mRNA for a cold shock protein gene in a cold shock vector. The recombinant expression vector constructed as described above is transferred into an appropriate host to prepare a transformant.

If a vector containing the above-mentioned chaperone-encoding gene is to be used in combination, the vector is further transferred to the transformant. If a cold shock vector has a gene encoding a chaperone, a host may be transformed with the vector alone.

The transformant is cultured under normal conditions. For example, cultivation may be conducted at about 37°C in case of Escherichia coli. The chaperone may be expressed in the host at all steps of cultivation, or the expression may be induced at the time of expression of the polypeptide of interest. Expression of the chaperone gene maybe induced depending on the state of existence of the chaperone gene in the host. For example, if expression of a chaperone gene inherent in the host is to be induced, the host may be placed under suitable conditions. If a gene encoding a chaperone is under the control of a heterogenous promoter (for example, in case where a chaperone gene inserted into a vector is transferred into a host), expression induction is conducted using a means suitable for the promoter controlling the transcription. If a host in which expression of a chaperone gene is constantly enhanced is to be used, the above-mentioned inductions process is unnecessary.

Expression of a polypeptide of interest is usually induced after the cell number is increased at a general cultivation temperature as described above. Cold shock response is induced in the host by lowering the cultivation temperature from the above state, resulting in preferential expression of the polypeptide of interest. Although it is not intended to limit the present invention, cold shock response is induced by shifting the cultivation temperature down to a temperature lower than a general cultivation temperature, for example, by 5°C or more, preferably 10°C or more. If a cold shock vector having an operator placed downstream of a promoter is used, the promoter may be induced by canceling the function of the operator using an appropriate means.
After the cold shock, cultivation of the transformant at a low temperature is further continued to express the polypeptide. The polypeptide of interest can be produced by collecting the polypeptide from the thus obtained culture. The polypeptide in the culture can be purified from the transformant cells collected from the culture or the culture supernatant, or both. Purification of the polypeptide may be conducted using a combination of known protein purification techniques such as ammonium sulfate fractionation, ultrafiltration and various chromatographies.

The purification can be facilitated by designing the expressed polypeptide in a form for binding to a carrier through an appropriate ligand. For example, a vector is designed so that a tag of about six histidine residues is attached on the N-terminal side of the polypeptide. Then, the resulting fusion polypeptide can bind to a metal (e.g., nickel)-chelate carrier via the histidine residues. The expressed polypeptide can be readily separated from host-derived proteins using such a carrier. Only the polypeptide of interest can be readily released from the carrier by cleaving, with a protease, the expressed polypeptide bound to the carrier at the linker. It is naturally possible to release the expressed polypeptide from the carrier as it is by elution using imidazole without cleavage. Besides the above-mentioned histidine tag, it may be possible to apply a method in which affinity purification is carried out using glutathione-S-transferase or a portion thereof as a tag and glutathione resin, a method in which purification is carried out using maltose-binding protein or a portion thereof as a tag and maltose resin, or the like.
In addition, affinity to an antibody may be utilized. The tag for purification may be designed to be located on either the N-terminal side or the C-terminal side of the expressed protein. Those skilled in the art would generally recognize the genetic engineering techniques and the affinity purification methods.

Since expression of polypeptides other than the polypeptide of interest is suppressed in a system for expressing a polypeptide using a cold shock vector as described above, the method of the present invention is advantageous for production of a highly pure polypeptide.

### Examples

The following Examples illustrate the present invention in more detail, but are not to be construed to limit the scope thereof.
Among the procedures described herein, basic procedures including preparation of plasmids and restriction enzyme digestion were carried out as described in J. Sambrook et al. (eds.), Molecular Cloning: A Laboratory Manual 3rd ed., Cold Spring Harbor Laboratory (2001).

### Example 1: Examination of expression of hDi-ASI by co-expression with chaperone

### (1) Construction of expression vector

An expression vector was constructed as follows in order to express a polypeptide consisting of PAZ + RNase III domain of human Dicer (679th to 1924th from the N terminus of the amino acid sequence of human Dicer).
First, synthetic primers 5 and 6 (SEQ ID NOS:4 and 5) were synthesized using a DNA synthesizer based on the nucleotide sequence available to the public under Genbank Acc No. AB028449, and purified according to a conventional method. The synthetic primer 5 is a synthetic DNA that has a recognition sequence for a restriction enzyme KpnI at nucleotide 9 to nucleotide 14, and a nucleotide sequence corresponding to amino acid 679 to amino acid 685 in the amino acid sequence of human Dicer (SEQ ID NO:3) at nucleotide 16 to nucleotide 36. The synthetic primer 6 has a recognition sequence for a restriction enzyme HindIII at nucleotide 9 to nucleotide 14 and a nucleotide sequence corresponding to amino acid 1919 to amino acid 1924 in the amino acid sequence of human Dicer (SEQ ID NO:3) at nucleotide 18 to nucleotide 35.

A PCR was conducted using the synthetic primers. The reaction conditions for the PCR were as follows.
Briefly, a reaction mixture of a total volume of 50 µl was prepared by adding 2 µl of a template DNA (human cDNA library, human pancreas, Takara Bio), 5 µl of 10 x LA PCR buffer (Takara Bio), 5 µl of dNTP mix (Takara Bio), 10 pmol of the synthetic primer 5, 10 pmol of the synthetic primer 6, 0.5 U of Takara LA Taq (Takara Bio) and sterile water. The reaction mixture was placed in TaKaRa PCR Thermal Cycler SP (Takara Bio) and subjected to a reaction as follows: 30 cycles of 94°C for 1 minute, 55°C for 1 minute and 72°C for 3 minutes.

After reaction, 5 µl of the reaction mixture was subjected to electrophoresis on 1.0% agarose gel. The observed about 2.7-kbp DNA fragment of interest was recovered and purified from the electrophoresis gel and subjected to ethanol precipitation. After ethanol precipitation, the recovered DNA was suspended in 5 µl of sterile water, and doubly digested with a restriction enzyme KpnI (Takara Bio) and a restriction enzyme HindIII (Takara Bio). The KpnI-HindIII digest was extracted and purified after electrophoresis on 1.0% agarose gel to obtain a KpnI-HindIII-digested DNA fragment.

Next, pCold08NC2 was constructed based on the description of WO 99/27117 using, as a starting material, a plasmid pMM047 harbored in Escherichia coli JM109/pMM047 (FERM BP-6523) (deposited on October 31, 1997 (date of original deposit) at International Patent Organism Depositary, National Institute of Advanced Science and Technology, AIST Tsukuba Central 6, 1-1, Higashi 1-chome, Tsukuba-shi, Ibaraki 305-8566, Japan). The plasmid pCold08NC2 has the following in this order from upstream to downstream: cspA promoter, lac operator, a modified Escherichia coli cspA gene-derived 5'-UTR and a multiple cloning site. In addition, the plasmid has the lacI gene, a downstream box sequence that is completely complementary to an anti-downstream sequence in the Escherichia coli 16S ribosomal RNA, a histidine tag consisting of six histidine residues, and a nucleotide sequence encoding an amino acid sequence recognized by factor Xa. The nucleotide sequence of the 5'-UTR region in the vector pCold08NC2 is shown in SEQ ID NO:2.

The vector pCold08NC2 was cleaved with the same restriction enzymes as those used upon preparation of the KpnI-HindIII-digested DNA fragment and the termini were dephosphorylated. The thus prepared vector and the KpnI-HindIII-digested DNA fragment were mixed together and ligated to each other using DNA ligation kit (Takara Bio). 20 µl of the ligation mixture was used to transform Escherichia coli JM109. Transformants were grown on LB medium containing agar at a concentration of 1.5%(w/v) and ampicillin at a concentration of 50 µg/ml.

A plasmid having the inserted DNA fragment of interest was confirmed by sequencing. This recombinant plasmid was designated as pCold08 hDi-ASI. This plasmid is designated and indicated as plasmid pCold08 hDi-ASI and has been deposited under accession number FERM BP-10076 at International Patent Organism Depositary, National Institute of Advanced Industrial Science and Technology, AIST Tsukuba Central 6, 1-1, Higashi 1-chome, Tsukuba-shi, Ibaraki 305-8566, Japan since September 26, 2003 (date of original deposit). pCold08 hDi-ASI is a plasmid containing a nucleotide sequence that encodes an amino acid sequence from amino acid 679 to amino acid 1924 (the nucleotide sequence of SEQ ID NO:6, the amino acid sequence of SEQ ID NO:7) in the amino acid sequence of human Dicer (SEQ ID NO:3). The protein expressed from the plasmid has a Perfect DB sequence, a His tag sequence and a factor Xa sequence. The amino acid sequence of the protein is shown in SEQ ID NO:8, and the nucleotide sequence is shown in SEQ ID NO:9.

### (2) Preparation of co-transformant

Escherichia coli BL21 was transformed with 1 ng of the above-mentioned plasmid pCold08/hDi-ASI and 1 ng of a chaperone plasmid pGro7 (which expresses GroEL and GroES), pKJE7 (which expresses DnaK, DnaJ and GrpE), pG-Tf2 (which expresses GroEL, GroES and Trigger Factor) or pTF16 (which expresses Trigger Factor) (all from Takara Bio) according to a calcium chloride method.

Co-transformants harboring pCold08/hDi-ASI and pGro7, pKJE7, pG-Tf2 or pTF16 were obtained by screening using plates containing chloramphenicol and ampicillin at concentrations of 20 µg/ml and 100 µg/ml, respectively. The thus obtained clones co-expressing hDi-ASI and one of the chaperones were designated as T1, T2, T3 and T4.

A transformant as a control was prepared by transforming Escherichia coli BL21 (Novagen) with pCold08/hDi-ASI alone and screening using a plate containing ampicillin at a concentration of 100 ug/ml. The clone for conventional expression system without the transfer of a chaperone was designated as C1.

### (3) Expression of hDi-ASI

Expression of hDi-ASI was examined using the respective transformants obtained in (1). 5 ml of LB liquid medium (containing 1% Bacto Tryptone, 0.5% yeast extract, 0.5% NaCl, 20 µg/ml chloramphenicol, 50 µg/ml ampicillin) was used for cultivation. A medium without chloramphenicol was used for culturing C1 as a control.

The respective transformants were cultured at 37°C. Upon initiation of the culture, expression of the chaperone was induced by adding L-arabinose at a final concentration of 0.5 mg/ml (for T1, T2 or T4) or tetracyclin at a final concentration of 5 ng/ml (for T3) to the medium. When the turbidity (OD600) reached about 0.4, cultivation was carried out at 15°C for 15 minutes, IPTG was added to the culture at a final concentration of 0.5 mM and cultivation was further carried out at 15°C for 24 hours to induce expression of hDi-ASI.

After inducing expression of hDi-ASI for 24 hours, cells were collected. The cells were disrupted by sonication to prepare a cell extract fraction. Then, a soluble fraction was separated from an insoluble fraction by centrifugation at 15,000 x g. Portion of the respective fractions each corresponding to about 3.75 x 10⁶ cells were subjected to SDS-PAGE. Results of analysis by CBB staining (A), and Western blotting using an anti-His tag antibody (Qiagen) (B) are shown in Figure 1.

As shown in Figure 1, the protein of interest having a molecular weight of 144000 was observed in the cell extract fraction of C1 as a control for conventional expression system, whereas almost no such a protein was observed in the soluble fraction. On the other hand, increase in hDi-ASI in the cell extract fraction as compared with the control was observed in case of co-expression of hDi-ASI with Trigger Factor as seen for T4. The protein was mainly detected in the soluble fraction. Almost no such a protein was observed in the soluble fraction in case of T1, T2 or T3 in which hDi-ASI was co-expressed with another chaperone.

As described above, it was shown that co-expression with Trigger Factor was effective in increasing expression level and solubility of hDi-ASI as compared with the conventional expression system without the transfer of a chaperone, or co-expression with chaperones expressing GroEL and GroES; Dnak, DnaJ and GrpE; or GroEL, GroES and Trigger Factor.

### (4) Expression and purification

Escherichia coli BL21 was transformed with pTf16 and pCold08 hDi-ASI prepared in (2) above, and transformants were grown on LB medium containing agar at a concentration of 1.5%(w/v) and ampicillin at a concentration of 50 µg/ml. A grown colony was inoculated into two vessels each containing 500 ml of TB liquid medium (6g of Bacto Tryptone, 12 g of Bacto Yeast Extract, 2 ml of glycerol, 17 mM KH₂PO₄, 72 mM K₂HPO₄, 25 mg of ampicillin) . Arabinose was added thereto at a final concentration of 0.5 mg/ml after inoculation. The cells were cultured at 37°C at 130 rpm until logarithmic growth phase, and then cooled to 15°C. After cooling, IPTG was added thereto at a final concentration of 1.0 mM, and the cells were cultured at 15°C at 130 rpm for 24 hours for expression induction. The cells were then collected by centrifugation to obtain 3.3 g of wet cells. 3.3 g of the wet cells were resuspended in 13.16 ml of binding buffer (50 mM tris-hydrochloride buffer (pH 8.5), 100 mM sodium chloride, 1 mM magnesium chloride, protease inhibitor (Complete, EDTA-free, Boehringer Mannheim)). The cells disrupted by sonication were subjected to centrifugation (12,000 rpm, 20 minutes) to separate a supernatant extract from a precipitate.
About 13 ml of the supernatant extract was further purified using a nickel column as follows.
Briefly, Ni-NTA agarose (Qiagen) corresponding to a resin volume of 10 ml was filled in a ϕ 50-mm column and washed with 30 ml of distilled water. The resin was then washed with 100 ml of binding buffer and collected. About 13 ml of the supernatant prepared from the cell disruption solution was added thereto and mixed gently at 4°C for about 1 hour using a rotary shaker. The resin to which the protein of interest had been adsorbed was filled in a ϕ 50-mm column and washed twice with 50 ml of binding buffer. The resin was then washed with 50 ml of buffer A (20 mM tris-hydrochloride buffer (pH 8.5), 100 mM sodium chloride, 1 mM magnesium chloride, 10% glycerol, 20 mM imidazole), 50 ml of buffer B (20 mM tris-hydrochloride buffer (pH 8.5), 800 mM sodium chloride, 1 mM magnesium chloride, 10% glycerol, 20 mM imidazole), and 50 ml of buffer A to remove unnecessary proteins other than the protein of interest.
After washing, elution was carried out with 30 ml of buffer C (20 mM tris-hydrochloride buffer (pH 8.5), 100 mM sodium chloride, 1 mM magnesium chloride, 10% glycerol, 100 mM imidazole). The eluted sample was concentrated using Centricon YM-10 (Amicon), 10 ml of buffer D (50 mM tris-hydrochloride buffer (pH 8.5), 250 mM sodium chloride, 1 mM magnesium chloride, 0.1 mM DTT, 0.1% Triton X-100, 10% glycerol) was added thereto, and the mixture was concentrated. This procedure was repeated twice. The concentrate was dialyzed against 500 ml of buffer E (50 mM tris-hydrochloride buffer (pH 8.5), 250 mM sodium chloride, 1 mM magnesium chloride, 0.1 mM DTT, 0.1% Triton X-100, 50% glycerol) to obtain about 220 µl of a protein sample. When a portion thereof was subjected to electrophoresis on 10% SDS-polyacrylamide, a band for the protein of interest was observed at a position corresponding to a molecular weight of about 144,000. This sample was used for activity determination below.

### (5) Measurement of dsRNA degradation activity

A dsRNA degradation activity of the protein sample prepared in (4) above was measured as follows.
First, a dsRNA as a substrate used for the activity measurements was synthesized using TurboScript T7 Transcription kit (GTS) according to the attached protocol.
Specifically, pDON-rsGFP was constructed by inserting a gene encoding red-shift green fluorescent protein (hereinafter referred to as GFP) (SEQ ID NO:10) from a plasmid pQBI1125 (Wako Pure Chemical Industries) into a plasmid pDON-AI (Takara Bio). A PCR was carried out using pDON-rsGFP as a template as well as a synthetic primer 3 (SEQ ID NO:11) which has a T7 promoter sequence and a synthetic primer 4 (SEQ ID NO: 12) to obtain an amplification product. An about 700-bp dsRNA was prepared by an RNA synthesis reaction using the resulting double-stranded DNA as a template and T7 RNA polymerase. A reaction mixture of a total volume of 10 µl was prepared by adding 1 µg of the dsRNA prepared above, 1 µl of the protein sample prepared in (3) above, 2 µl of 5 x reaction buffer (100 mM tris-hydrochloride buffer (pH 8.5), 750 mM sodium chloride, 12.5 mM magnesium chloride) and nuclease-free water. After the reaction mixture was reacted at 37°C for 18 hours, 10 µl of the reaction mixture was subjected to electrophoresis on 15% polyacrylamide gel. After electrophoresis, the gel was stained with ethidium bromide to observe the cleavage product. As a result, a degradation product of about 21 base pairs was observed, indicating its dsRNA degradation activity.
As described above, it was shown that a protein having a dsRNA degradation activity was expressed according to the method of the present invention.

### Example 2: Examination of expression of RTaseα and RTaseβ

Expression of a protein of interest alone, co-expression of a protein of interest with Trigger Factor and expression of a fusion protein of a protein of interest and Trigger Factor were compared with each other as follows. Two expression systems; i.e., a system in which a cold shock vector is used (cold shock expression system) and an expression system in which a combination of T7 promoter and T7 RNA polymerase is used (T7 promoter expression system) were used for expression of a fusion protein.

### (1) Construction of plasmid vectors

Synthetic primers TFN and TFCP (SEQ ID NOS:13 and 14) were synthesized using a DNA synthesizer based on the Escherichia coli Trigger Factor gene sequence (Genbank Acc. No. NC_000913, position 454357 to position 455655), and purified according to a conventional method.
The synthetic primer TFN is a synthetic DNA that has a nucleotide sequence encoding the 1st to 9th amino acids from the N terminus of the amino acid sequence of Escherichia coli Trigger Factor and a recognition sequence for a restriction enzyme NdeI at nucleotide 4 to nucleotide 9. The synthetic primer TFCP is a synthetic DNA that has a nucleotide sequence complementary to a nucleotide sequence encoding the 1st to 9th amino acids from the C terminus of the amino acid sequence of Escherichia coli Trigger Factor, a nucleotide sequence complementary to a nucleotide sequence encoding a recognition sequence for a protease factor Xa, a recognition sequence for a restriction enzyme EcoRI, a recognition sequence for a restriction enzyme BamHI, and a recognition sequence for a restriction enzyme HindIII. A genomic DNA as a template for PCR was extracted from Escherichia coli HB101 (Takara Bio).

A PCR was conducted using the synthetic primers and the genomic DNA. The reaction conditions for the PCR were as follows. Briefly, a reaction mixture of a total volume of 100 µl was prepared by adding 1 µl of the template DNA prepared as described above, 10 µl of 10 x Pyrobest buffer II (Takara Bio), 8 µl of dNTP mix (Takara Bio), 100 pmol of the synthetic primer TFN, 100 pmol of the synthetic primer TFCP, 2.5 U of Pyrobest DNA polymerase (Takara Bio) and sterile water. The reaction mixture was placed in PCR Thermal Cycler SP (Takara Bio) and subjected to a reaction as follows: 30 cycles of 94°C for 30 seconds, 59°C for 30 seconds and 72°C for 2 minutes.

After reaction, 100 µl of the reaction mixture was subjected to electrophoresis on 1% agarose gel. The observed about 1.5-kbp DNA fragment of interest was recovered and purified from the electrophoresis gel and subjected to ethanol precipitation. After ethanol precipitation, the recovered DNA was suspended in 15 µl of sterile water, and doubly digested with a restriction enzyme NdeI (Takara Bio) and a restriction enzyme HindIII (Takara Bio). The NdeI-HindIII digest was extracted and purified after electrophoresis on 1% agarose gel to obtain an NdeI-HindIII-digested DNA fragment.

Next, a plasmid vector pColdII (Takara Bio) was doubly digested with restriction enzymes NdeI and HindIII, and the termini were dephosphorylated. The thus prepared vector and the NdeI-HindIII-digested DNA fragment were mixed together and ligated to each other using DNA ligation kit (Takara Bio). 10 µl of the ligation mixture was used to transform Escherichia coli JM109. Transformants were grown on LB medium containing agar at a concentration of 1.5%(w/v) and ampicillin at a concentration of 100 µg/ml. A plasmid having the inserted DNA fragment of interest was confirmed by sequencing. Subsequently, a silent mutation was introduced in order to eliminate a recognition sequence for a restriction enzyme EcoRI in the Trigger Factor gene sequence in the plasmid (Genbank Acc. No. NC_000913, position 455107 to position 455112). The thus obtained recombinant plasmid which has cold shock expression system and contains Escherichia coli Trigger Factor gene.sequence was designated as pColdTF.

A plasmid vector for expressing a fusion protein of a protein of interest and Escherichia coli Trigger Factor using T7 promoter expression system was constructed as follows.

First, pColdTF was doubly digested with a restriction enzyme EcoRI (Takara Bio) and a restriction enzyme Eco0109I (Takara Bio), and the termini were dephosphorylated to obtain an EcoRI-Eco0109I-digested DNA fragment. Next, pCold08NC2 was doubly digested with a restriction enzyme EcoRI and a restriction enzyme EcoO109I and subjected to electrophoresis on 1% agarose gel. An EcoRI-EcoO109I digest was extracted and purified, and mixed and ligated with the EcoRI-EcoO109I-dogested DNA fragment using DNA ligation kit (Takara Bio). 10 µl of the ligation mixture was used to transform Escherichia coli JM109. Transformants were grown on LB medium containing agar at a concentration of 1.5%(w/v) and ampicillin at a concentration of 100 µg/ml. The thus obtained recombinant plasmid in which a multiple cloning site in pColdTF had been modified was designated as pColdTF-II.

pColdTF-II was digested with a restriction enzyme XbaI (Takara Bio), blunted with T4 DNA polymerase (Takara Bio) and then digested with a restriction enzyme NdeI to obtain an NdeI-blunt end fragment which contains Escherichia coli Trigger Factor gene.

A plasmid vector pET16b (Novagen) was digested with a restriction enzyme BamHI (Takara Bio), blunted with T4 DNA polymerase and then digested with a restriction enzyme NdeI, and the termini were dephosphorylated. The thus prepared vector and the NdeI-blunt end DNA fragment containing Escherichia coli Trigger Factor gene were mixed together and ligated to each other using DNA ligation kit. 10 µl of the ligation mixture was used to transform Escherichia coli JM109. Transformants were grown on LB medium containing agar at a concentration of 1.5%(w/v) and ampicillin at a concentration of 100 µg/ml. The thus obtained recombinant plasmid which has a T7 promoter expression system and contains Escherichia coli Trigger Factor gene sequence was designated as pETTF.

### (2) Construction of vectors for expressing RTaseα and RTaseβ

A double-stranded DNA having a sequence of SEQ ID NO:15 was synthesized based on the amino acid sequence of Rous associated virus 2 (RAV-2) reverse transcriptase α subunit (hereinafter referred to as RAV-2 RTaseα) (Genbank Acc. No. BAA22090, 1st to 572nd amino acids from the N terminus). The nucleotide sequence was modified according to the codon usage of Escherichia coli without altering the encoded amino acid sequence, and designed to have a recognition sequence for a restriction enzyme EcoRI and a recognition sequence for a restriction enzyme XbaI at both ends.

A double-stranded DNA having a sequence of SEQ ID NO:16 was synthesized based on the amino acid sequence of Rous associated virus 2 (RAV-2) reverse transcriptase β subunit (hereinafter referred to as RAV-2 RTaseβ) (Genbank Acc. No. BAA22090). The nucleotide sequence was modified according to the codon usage of Escherichia coli without altering the encoded amino acid sequence, and designed to have a recognition sequence for a restriction enzyme EcoRI and a recognition sequence for a restriction enzyme XbaI at both ends.

The two synthetic double-stranded DNAs were doubly digested with restriction enzymes EcoRI (Takara Bio) and XbaI (Takara Bio), and subjected to electrophoresis on 1% agarose gel. The observed DNA fragments of the sizes of interest were recovered and purified from the electrophoresis gel to obtain an EcoRI-XbaI-digested DNA fragment containing a RAV-2 RTaseα-encoding gene and an EcoRI-XbaI-digested DNA fragment containing a RAV-2 RTaseβ-encoding gene.

pColdTF prepared in (1) was doubly digested with restriction enzymes EcoRI and XbaI, and the termini were dephosphorylated. The thus prepared vector and one of the two EcoRI-XbaI-digested DNA fragments were mixed together and ligated to each other using DNA ligation kit (Takara Bio). 10 µl of the ligation mixture was used to transform Escherichia coli JM109. Transformants were grown on LB medium containing agar at a concentration of 1.5%(w/v) and ampicillin at a concentration of 100 µg/ml. The plasmids for expressing a fusion protein of Trigger Factor and RAV-2 RTaseα and a fusion protein of Trigger Factor and RAV-2 RTaseβ in cold shock expression system were designated as pColdTF-α and pColdTF-β, respectively.

Furthermore, a plasmid for expressing RAV-2 RTaseα alone and a plasmid for expressing RAV-2 RTaseβ alone were prepared according to the method as described for pColdTF-α and pColdTF-β except pCold08Nc2 constructed in Example 1 was used in place of pColdTF. The prepared plasmids were designated as pCold08-α and pCold08-β, respectively.

Plasmids for expressing a fusion protein of Trigger Factor and RAV-2 RTaseα and a fusion protein of Trigger Factor and RAV-2 RTaseβ in T7 promoter expression system were constructed as follows.

pColdTF-α and pColdTF-β were digested with a restriction enzyme XbaI (Takara Bio), blunted with T4 DNA polymerase (Takara Bio), then digested with a restriction enzyme EcoRI, and subjected to electrophoresis on 1% agarose gel. The observed DNA fragments of the sizes of interest were recovered and purified from the electrophoresis gel to obtain an EcoRI-blunt end DNA fragment containing a gene encoding RAV-2 RTaseα and an EcoRI-blunt end DNA fragment containing a gene encoding RAV-2 RTaseβ.

pETTF prepared in (1) was digested with a restriction enzyme SalI (Takara Bio), blunted with T4 DNA polymerase and then digested with a restriction enzyme EcoRI, and the termini were dephosphorylated. The thus prepared vector and one of the two EcoRI-blunt end DNA fragments were mixed together and ligated to each other using DNA ligation kit. 10 µl of the ligation mixture was used to transform Escherichia coli JM109. Transformants were grown on LB medium containing agar at a concentration of 1.5%(w/v) and ampicillin at a concentration of 100 µg/ml. The plasmids for expressing a fusion protein of Trigger Factor and RAV-2 RTaseα and a fusion protein of Trigger Factor and RAV-2 RTaseβ in T7 promoter expression system were designated as pETTF-α and pETTF-β, respectively.

### (3) Preparation of transformants

pColdTF-α for expressing a fusion protein of Trigger Factor and RAV-2 RTaseα in cold shock expression system was used to transform Escherichia coli BL21 according to a calcium chloride method. A transformant was obtained by screening using a plate containing ampicillin at a concentration of 100 µg/ml.

A transformant of Escherichia coli BL21 transformed with pColdTF-β for expressing a fusion protein of Trigger Factor and RAV-2 RTaseβ in cold shock expression system was also prepared in a similar manner.
The following transformants were also prepared for comparison with the above-mentioned fusion expression system: transformants for expressing RAV-2 RTaseα or RAV-2 RTaseβ in sole expression system; a transformant for expressing RAV-2 RTaseα and Trigger Factor in co-expression system; a transformant for expressing RAV-2 RTaseβ and Trigger Factor in co-expression system; a transformant for expressing a fusion protein of Trigger Factor and RAV-2 RTaseα in T7 promoter expression system; and a transformant for expressing a fusion protein of Trigger Factor and RAV-2 RTaseβ in T7 promoter expression system.

Transformants for expression in sole expression system were obtained according to a preparation method similar to that described above with respect to preparation of transformants for expressing fusion proteins in cold shock expression system except that BL21 was transformed with the plasmid pCold08-α or pCold08-β.

Transformants for expression in co-expression system were prepared as follows. First, the plasmid pTf16 and the plasmid pCold08-α or pCold08-β were used to transform Escherichia coli BL21 according to a calcium chloride method. Co-transformants harboring the plasmid pTf16 and pCold08-α or pCold08-β were obtained by screening using plates containing chloramphenicol and ampicillin at concentrations of 100 µg/ml and 20 µg/ml, respectively.

Transformants for expressing fusion proteins in T7 promoter expression system were obtained according to a preparation method similar to that described above with respect to preparation of transformants for expressing fusion proteins in cold shock expression system except that the plasmid pETTF-α or pETTF-β was used, and BL21(DE3) (Novagen) was subjected to transformation.

### (4) Expression of RTaseα and RTaseβ

Expression of RTaseα and RTaseβ was examined using the transformants obtained in (3). The transformants for expressing fusion proteins in cold shock expression system and the transformants for sole expression system were cultured using 5 ml of LB liquid medium containing ampicillin at a concentration of 50 µg/ml. The transformants for co-expression system were cultured using 5 ml of LB liquid medium containing ampicillin, chloramphenicol and arabinose at concentrations of 50 µg/ml, 20 µg/ml and 0.5 mg/ml, respectively. The respective transformants were cultured at 37°C. When the turbidity (OD600) reached about 0.4, cultivation was carried out at 15°C for 15 minutes, IPTG was added to the culture at a final concentration of 1 mM, and cultivation was carried out at 15°C for 24 hours for expression induction. After expression induction for 24 hours, cells were collected.

The transformants for expressing fusion proteins in T7 promoter expression system were cultured using 5. ml of LB liquid medium containing ampicillin at a concentration of 50 µg/ml. The transformants were cultured at 37°C. When the turbidity (OD600) reached about 0.4, IPTG was added to the culture at a final concentration of 1 mM, and cultivation was carried out at 37°C for 3 hours for expression induction. After expression induction for 3 hours, cells were collected.

The cells were suspended in PBS and disrupted by sonication to prepare a cell extract fraction. Then, a soluble fraction was separated from an insoluble fraction by centrifugation at 15,000 x g. Portion of the respective fractions each corresponding to about 3.75 x 10⁶ cells were subjected to SDS-PAGE. Results of analysis by CBB staining are shown in Figure 2.

As shown in Figure 2, in cases of sole expression of the proteins of interest using pCold08-α or pCold08-β (A) and co-expression of the proteins of interest with Trigger Factor using pCold08-α and pTf16, or pCold08-β and pTf16 (B), expression products corresponding to the molecular weights 63,000 Da and 98,000 Da of RAV-2 RTaseα and RAV-2 RTaseβ, respectively, were observed in the cell extract fractions, but almost no such expression product was observed in the soluble fractions. In cases of expression of the fusion proteins of the proteins of interest and Trigger Factor using pETTF-α or pETTF-β in T7 promoter expression system (C), the majority of the proteins of interest detected in the cell extract fractions was detected in the insoluble fractions. On the other hand, in cases of fusion expression of the proteins of interest and Trigger Factor using pColdTF-α or pColdTF-β (D), the majority of the proteins of interest detected in the cell extract fractions was detected in the soluble fractions.

### Example 3: Expression of DNase

### (1) Construction of vector for expressing DNase

pCold08-End1 (FERM BP-10313) (deposited on February 16, 2005 (date of original deposit) at International Patent Organism Depositary, National Institute of Advanced Science and Technology, AIST Tsukuba Central 6, 1-1, Higashi 1-chome, Tsukuba-shi, Ibaraki 305-8566, Japan) was used as a plasmid for expressing DNase alone. This plasmid contains a nucleotide sequence encoding a DNase consisting of 254 amino acid residues and is constructed so as to express a fusion protein of 271 amino acid residues in which His-Tag, a recognition sequence for factor Xa and a linker sequence are added to the DNase.

A plasmid for expressing a fusion protein of Trigger Factor and DNase was constructed as follows.
First, synthetic primers NUCN and NUCC (SEQ ID NOS:17 and 18) were synthesized using a DNA synthesizer based on the nucleotide sequence of pCold08-End1, and purified according to a conventional method. The synthetic primer NUCN is a synthetic DNA that has a nucleotide sequence encoding the 1st to 7th amino acids from the N terminus of DNase and a recognition sequence for a restriction enzyme EcoRI at nucleotide 4 to nucleotide 9. The synthetic primer NUCC is a synthetic DNA that has a nucleotide sequence complementary to a nucleotide sequence encoding the 247th to 254th amino acids from the N terminus of DNase and a recognition sequence for a restriction enzyme BamHI at nucleotide 4 to nucleotide 9.

A PCR was conducted using the synthetic primers. The reaction conditions for the PCR were as follows. Briefly, a reaction mixture of a total volume of 100 µl was prepared by adding 1 µl of a template DNA (pCold08-End1), 10 µl of 10 x Pyrobest buffer II (Takara Bio), 8 µl of dNTP mix (Takara Bio), 100 pmol of the synthetic primer NUCN, 100 pmol of the synthetic primer NUCC, 2.5 U of Pyrobest DNA polymerase (Takara Bio) and sterile water. The reaction mixture was placed in PCR Thermal Cycler SP (Takara Bio) and subjected to a reaction as follows: 30 cycles of 94°C for 30 seconds, 58°C for 30 seconds and 72°C for 1 minute.

After reaction, 100 µl of the reaction mixture was subjected to electrophoresis on 1% agarose gel. The observed about 0.8-kbp DNA fragment of interest was recovered and purified from the electrophoresis gel and subjected to ethanol precipitation. After ethanol precipitation, the recovered DNA was suspended in 15 µl of sterile water, and doubly digested with a restriction enzyme EcoRI (Takara Bio) and a restriction enzyme BamHI (Takara Bio). The EcoRI-BamHI digest was extracted and purified after electrophoresis on 1% agarose gel to obtain an EcoRI-BamHI-digested DNA fragment.

Next, pColdTF prepared in Example 2(2) was doubly digested with restriction enzymes EcoRI and BamHI, and the termini were dephosphorylated. The thus prepared vector and one of the two EcoRI-BamHI-digested DNA fragments were mixed together and ligated to each other using DNA ligation kit (Takara Bio). 10 µl of the ligation mixture was used to transform Escherichia coli JM109. Transformants were grown on LB medium containing agar at a concentration of 1.5%(w/v) and ampicillin at a concentration of 100 µg/ml. A plasmid having the inserted DNA fragment of interest was prepared. The plasmid for expressing a fusion protein of Trigger Factor and DNase was designated as pColdTF-End1.

### (2) Preparation of transformant

pColdTF-End1 for expressing a fusion protein of Trigger Factor and DNase was used to transform Escherichia coli BL21 according to a calcium chloride method. A transformant was obtained by screening using a plate containing ampicillin at a concentration of 100 µg/ml.

A transformant for expressing DNase in sole expression system and a transformant for expressing DNase and Trigger Factor in co-expression system were also prepared for comparison with the fusion expression system.

A transformant for expression in sole expression system was obtained according to a preparation method similar to that of the above-mentioned transformant except that BL21 was transformed with the plasmid pCold08-End1.
A transformant for expression in co-expression system was prepared as follows. First, the plasmid pCold08-End1 and the plasmid pTf16 were used to transform Escherichia coli BL21 according to a calcium chloride method. A co-transformant harboring the plasmids pCold08-End1 and pTf16 was obtained by screening using plates containing chloramphenicol and ampicillin at concentrations of 100 µg/ml and 20 µg/ml, respectively.

### (3) Expression of DNase

Expression of DNase was examined using the transformants obtained in (2). The transformant for fusion expression system and the transformant for sole expression system were cultured using 5 ml of LB liquid medium containing ampicillin at a concentration of 50 µg/ml. The transformant for co-expression system was cultured using 5 ml of LB liquid medium containing ampicillin, chloramphenicol and arabinose at concentrations of 50 µg/ml, 20 µg/ml and 0.5 mg/ml, respectively. The respective transformants were cultured at 37°C. When the turbidity (OD600) reached about 0.8, cultivation was carried out at 15°C for 15 minutes, IPTG was added to the culture at a final concentration of 1 mM, and cultivation was carried out at 15°C for 24 hours for expression induction. After expression induction for 24 hours, cells were collected. The cells were suspended in PBS and disrupted by sonication to prepare a cell extract fraction. Then, a soluble fraction was separated from an insoluble fraction by centrifugation at 15,000 x g. Portion of the respective fractions each corresponding to 0.05 OD (OD600) were subjected to SDS-PAGE (5-20% gel). Results of analysis by CBB staining are shown in Figure 3.

As shown in Figure 3, in cases of sole expression of the protein of interest using pCold08-End1 (A) and co-expression of the protein of interest with Trigger Factor using pCold08-End1 and pTf16 (B), no distinct band for an expression product corresponding to the molecular weight 31,000 Da of DNase was observed after CBB staining for the soluble fraction or the insoluble fraction. In case of fusion expression of DNase and Trigger Factor using pColdTF-End1 (C), a band corresponding to the protein of interest was detected for the soluble fraction.

### (4) Measurement of DNase activity

A DNase activity of the sonication soluble fraction of fusion expression system prepared in (3) was measured. A sonication soluble fraction from Escherichia coli transformed with the vector pColdTF (without an incorporated insert) alone was also obtained as a control and the activity was measured at the same time. The sonication soluble fraction as a control was obtained in a manner similar to that in (2) and (3) above except that pColdTF was used.
λ-HindIII digest (Takara Bio) was used as a substrate for activity measurements. A reaction mixture of a total volume of 50 µl was prepared by adding 1 µg of λ-HindIII digest, the sonication soluble fraction corresponding to 0.025 OD (OD600), 5 µl of 10 x reaction buffer (400 mM tris-hydrochloride buffer (pH 7.5), 100 mM sodium chloride, 60 mM magnesium chloride, 10 mM calcium chloride) and nuclease-free water. The reaction mixture was reacted at 20°C for 2 hours. 10 µl of the reaction mixture was subjected to electrophoresis on 1% agarose gel for analyzing a cleavage product. The results are shown in Figure 4.
As shown in Figure 4, almost no degradation of the substrate was observed when the sonication soluble fraction as a control was used (lane 1). On the other hand, the substrate was degraded and a DNase activity was observed when the sonication soluble fraction of the fusion expression system of DNase and Trigger Factor was used (lane 2).

### Example 4: Examination of expression of hDi-PAZ

### (1) Construction of expression vector

An expression vector was constructed as follows in order to express a polypeptide (892nd to 1064th from the N terminus of the amino acid sequence of human Dicer; also referred to as PAZ) which contains PAZ domain of human Dicer (895th to 1064th from the N terminus of the amino acid sequence of human Dicer).
First, synthetic primers 1 and 2 (SEQ ID NOS:19 and 20) were synthesized using a DNA synthesizer based on the nucleotide sequence available to the public under Genbank Acc. No. AB028449, and purified according to a conventional method. The synthetic primer 1 is a synthetic DNA that has a recognition sequence for a restriction enzyme KpnI at nucleotide 9 to nucleotide 14, and a nucleotide sequence corresponding to amino acid 892 to amino acid 898 in the amino acid sequence of human Dicer (SEQ ID NO:3) at nucleotide 16 to nucleotide 36. The synthetic primer 2 has a recognition sequence for a restriction enzyme HindIII at nucleotide 9 to nucleotide 14 and a nucleotide sequence corresponding to amino acid 1058 to amino acid 1064 in the amino acid sequence of human Dicer (SEQ ID NO:3) at nucleotide 15 to nucleotide 36.

A PCR was conducted using the synthetic primers. pCold08/hDi-ASI prepared in Example 1-(2) was used as a template DNA. The reaction conditions for the PCR were as follows.
Briefly, a reaction mixture of a total volume of 100 µl was prepared by adding 1 ng of the template DNA, 10 µl of 1.0 x LA PCR buffer (Takara Bio), 8 µl of dNTP mix (Takara Bio), 10 pmol of the synthetic primer 5, 10 pmol of the synthetic primer 6, 0.5 U of Takara Ex Taq (Takara Bio) and sterile water. The reaction mixture was placed in TaKaRa PCR Thermal Cycler MP (Takara Bio) and subjected to a reaction as follows: 30 cycles of 94°C for 30 seconds, 55°C for 30 seconds and 72°C for 2 minutes.

After reaction, 95 µl of the reaction mixture was subjected to electrophoresis on 1.0% agarose gel. The observed about 530-bp DNA fragment of interest was recovered and purified from the electrophoresis gel and subjected to ethanol precipitation. After ethanol precipitation, the recovered DNA was suspended in 5 µl of sterile water, and doubly digested with a restriction enzyme KpnI (Takara Bio) and a restriction enzyme HindIII (Takara Bio). The KpnI-HindIII digest was extracted and purified after electrophoresis on 1.0% agarose gel to obtain a KpnI-HindIII-digested DNA fragment.

The vector pCold08NC2 was cleaved with the same restriction enzymes as those used upon preparation of the KpnI-HindIII-digested DNA fragment, and the termini were dephosphorylated. The thus prepared vector and the KpnI-HindIII-digested DNA fragment were mixed together and ligated to each other using DNA ligation kit (Takara Bio). 6 µl of the ligation mixture was used to transform Escherichia coli JM109. Transformants were grown on LB medium containing agar at a concentration of 1.5%(w/v) and ampicillin at a concentration of 100 µg/ml.

A plasmid having the inserted DNA fragment of interest was designated as pCold08/hDi-PAZ. pCold08/hDi-PAZ is a plasmid containing a nucleotide sequence that encodes an amino acid sequence from amino acid 892 to amino acid 1064 in the amino acid sequence of human Dicer (SEQ ID NO:3). The protein expressed from the plasmid has a Perfect DB sequence, a His tag sequence and a factor-Xa sequence.

A plasmid for expressing a fusion protein of Trigger Factor and PAZ was prepared according to a preparation method similar to that described above with respect to preparation of pCold08/hDi-PAZ except that pColdTF-II prepared in Example 2-(1) was used as a vector.
This plasmid was designated as pColdTF/hDi-PAZ.

### (2) Preparation of transformant

pColdTF/hDi-PAZ for expressing a fusion protein of Trigger Factor and hDi-PAZ was used to transform Escherichia coli BL21 according to a calcium chloride method. A transformant was obtained by screening using a plate containing ampicillin at a concentration of 100 µg/ml.

A transformant for expressing hDi-PAZ in sole expression system and a transformant for expressing hDi-PAZ and Trigger Factor in co-expression system were also prepared for comparison with the fusion expression system.

A transformant for expression in sole expression system was obtained according to a preparation method similar to that of the transformant for expression in fusion expression system except that BL21 was transformed with the plasmid pCold08/hDi-PAZ. A transformant for expression in co-expression system was prepared as follows.
First, the plasmid pCold08/hDi-PAZ and the plasmid pTf16 were used to transform Escherichia coli A19 according to a calcium chloride method. A co-transformant harboring the plasmids pCold08/hDi-PAZ and pTf16 was obtained by screening using plates containing chloramphenicol and ampicillin at concentrations of 50 µg/ml and 100 µg/ml, respectively.

### (3) Expression of hDi-PAZ

Expression of hDi-PAZ was examined using the transformants obtained in (2). The transformant for fusion expression system and the transformant for sole expression system were cultured using 3 ml of LB liquid medium containing ampicillin at a concentration of 50 µg/ml. The transformant for co-expression system was cultured using 3 ml of LB liquid medium containing ampicillin, chloramphenicol and arabinose at concentrations of 50 µg/ml, 20 µg/ml and 0.5 mg/ml, respectively. The respective transformants were cultured at 37°C. When the turbidity (OD600) reached about 0.4, cultivation was carried out at 15°C for 15 minutes, IPTG was added to the culture at a final concentration of 0.5 mM, and cultivation was carried out at 15°C for 24 hours for expression induction. After expression induction for 24 hours, cells were collected. The cells were suspended in cell disruption solution (50 mM tris-HCl (pH 8.5), 100 mM NaCl, 1 mM MgCl₂, protease inhibitor (complete EDTA-Free)) and disrupted by sonication to prepare a cell extract fraction. Then, a soluble fraction was separated from an insoluble fraction by centrifugation at 15,000 x g. Portion of the respective fractions each corresponding to about 2.5 x 10⁶ cells were subjected to SDS-PAGE. Analysis was carried out by CBB staining.

As a result, in cases of sole expression of the protein of interest using pCold08/hDi-PAZ and co-expression of the protein of interest with Trigger Factor using pCold08/hDi-PAZ and pTf16, an expression product corresponding to the molecular weight 24,000 Da of the protein of interest was observed in the cell extract fraction, whereas almost no such an expression product was detected in the soluble fraction. In case of fusion expression of the protein of interest and Trigger Factor using pColdTF/hDi-PAZ, the amount of the protein of interest in the cell extract fraction was increased as compared with the control. The majority was detected in the soluble fraction.

### Industrial Applicability

According to the method of the present invention, it is possible to produce a considerable amount of a polypeptide of interest with high purity while retaining its activity.

### Sequence Listing Free Text

SEQ ID NO:2; A gene encoding mutated 5'-UTR of Escherichia coli cspA gene
SEQ ID NO:4; Synthetic primer 5 to amplify a gene encoding human dicer mutant
SEQ ID NO:5; Synthetic primer 6 to amplify a gene encoding human dicer mutant
SEQ ID NO:6; A gene encoding human dicer mutant
SEQ ID NO:7; An amino acid sequence of human dicer mutant
SEQ ID NO:8; An amino acid sequence of human dicer mutant
SEQ ID NO:9; A gene encoding human dicer mutant
SEQ ID NO:10; A gene encoding red-shift green fluorescent protein.
SEQ ID NO:11; Synthetic primer 3 to amplify a gene encoding red-shift green fluorescent protein
SEQ ID NO:12; Synthetic primer 4 to amplify a gene encoding red-shift green fluorescent protein
SEQ ID NO:13; Synthetic primer TFN to amplify a gene encoding Trigger Factor
SEQ ID NO:14; Synthetic primer TFCP to amplify a gene encoding Trigger Factor
SEQ ID NO:15; A gene encoding RAV-2 reverse transcriptase alpha subunit
SEQ ID NO:16; A gene encoding RAV-2 reverse transcriptase beta subunit
SEQ ID NO:17; Synthetic primer NUCN to amplify a gene encoding DNase
SEQ ID NO:18; Synthetic primer NUCC to amplify a gene encoding DNase
SEQ ID NO:19; Synthetic primer 1 to amplify a gene encoding human dicer PAZ domain
SEQ ID NO:20; Synthetic primer 2 to amplify a gene encoding human dicer PAZ domain

## Claims

1. A method for producing a polypeptide, the method comprising exposing a host having a gene encoding a desired polypeptide being transferred into the host to low-temperature conditions to induce expression of the polypeptide, wherein expression of a gene encoding a chaperone is enhanced in the host.

2. The method according to claim 1, wherein expression of the gene encoding a chaperone is enhanced by one selected from the group consisting of: induction of expression of a chaperone gene of the host; modification of a chaperone gene on a chromosome of the host; transfer of a chaperone gene into the host; and use of a host in which expression of a chaperone gene is enhanced.

3. The method according to claim 1, wherein the chaperone is selected from the group consisting of DnaK, DnaJ, GrpE, GroEL, GroES and Trigger Factor.

4. The method according to claim 1, wherein the gene encoding a desired polypeptide being transferred into the host is linked downstream of a DNA encoding a 5'-untranslated region derived from an mRNA for a cold shock protein gene.

5. The method according to claim 4, wherein the gene encoding a desired polypeptide being transferred into the host is linked downstream of a DNA encoding a 5'-untranslated region derived from an mRNA for Escherichia coli cspA gene.

6. The method according to claim 1, wherein the gene encoding a desired polypeptide and the gene encoding a chaperone are transferred into the host using vector(s).

7. The method according to claim 6, wherein the gene encoding a desired polypeptide and the gene encoding a chaperone are linked to each other so that a fusion protein of the desired polypeptide and the chaperone is encoded.

8. The method according to claim 7, wherein the desired polypeptide is selected from the group consisting of RAV-2 reverse transcriptase α subunit, RAV-2 reverse transcriptase β subunit, DNase and human Dicer PAZ domain polypeptide.

9. The method according to claim 1, wherein the host is Escherichia coli.

10. A set of plasmid vectors used for production of a desired polypeptide, comprising:
(1) a first vector having, downstream of a promoter:
(a) a DNA encoding a 5'-untranslated region derived from an mRNA for a cold shock protein gene; and
(b) a restriction enzyme recognition sequence that can be used for inserting a gene encoding a desired polypeptide and is located downstream of the DNA of (a); and
(2) a second vector having a gene encoding a chaperone,
wherein replication origins of the vectors of (1) and (2) are selected so that incompatibility is not exerted.

11. The set of vectors according to claim 10,
wherein the first vector contains a DNA encoding a 5'-untranslated region derived from an mRNA for Escherichia coli cspA gene.

12. The set of vectors according to claim 10,
wherein the second vector contains a gene encoding a chaperone selected from the group consisting of DnaK, DnaJ, GrpE, GroEL, GroES and Trigger Factor.

13. The set of vectors according to claim 10, which consists of plasmids that are capable of replicating in Escherichia coli.

14. An expression vector having, downstream of a promoter:
(a) a DNA encoding a 5'-untranslated region derived from an mRNA for a cold shock protein gene;
(b) a DNA having a restriction enzyme recognition sequence that can be used for inserting a gene encoding a desired polypeptide and is located downstream of the DNA of (a); and
(c) a gene encoding a chaperone.

15. The expression vector according to claim 14, which contains a DNA encoding a 5'-untranslated region derived from an mRNA for Escherichia coli cspA gene.

16. The expression vector according to claim 14, which is a plasmid containing a gene encoding a chaperone selected from the group consisting of DnaK, DnaJ, GrpE, GroEL, GroES and Trigger Factor.

17. The expression vector according to claim 14,
wherein the restriction enzyme recognition sequence that can be used for inserting a gene encoding a desired polypeptide is located at a position at which the gene encoding a desired polypeptide can be inserted so that the desired polypeptide is expressed as a fusion protein with the chaperone.

18. The expression vector according to claim 14, which is a plasmid capable of replicating in Escherichia coli.
